# EUROPEAN PATENT APPLICATION

(11) **EP 3 753 559 A1**
(43) Date of publication of application: **23.12.2020**
(21) Application number: 19754426.5
(22) Date of filing: 12.02.2019
(51) Int. Cl.: A61K 31/22, A61P 1/04

(54) **COLITIS IMPROVING AGENT**

(30) Priority: 13.02.2018 JP 2018023548
(71) Applicant: Asta Pharmaceuticals Co., Ltd., Nakaniikawa-gun Toyama 930-0397 (JP)
(72) Inventor: TAKASHINA, Michinori, Funabashi-shi, Chiba 273-0002 (JP); OGAWA, Makoto, Nakaniikawa-gun, Toyama 930-0397 (JP); KOBAYASHI, Kaoru, Mizuho-shi, Gifu 501-0307 (JP); NISHIDA, Yasuhiro, Nakaniikawa-gun, Toyama 930-0397 (JP); NAKAGAWA, Akiko, Nakaniikawa-gun, Toyama 930-0397 (JP); FUJITA, Takashi, Nakaniikawa-gun, Toyama 930-0397 (JP); KOBAYASHI, Satoshi, Nakaniikawa-gun, Toyama 930-0397 (JP); SHINOHARA, Ryoma, Nakaniikawa-gun, Toyama 930-0397 (JP); IWANO, Yuji, Nakaniikawa-gun, Toyama 930-0397 (JP); MORIIZUMI, Kiyotaka, Nakaniikawa-gun, Toyama 930-0397 (JP); MAEDA, Sachi, Nakaniikawa-gun, Toyama 930-0397 (JP); SAKATA, Ryo, Nakaniikawa-gun, Toyama 930-0397 (JP); YOKOYAMA, Tomihisa, Nakaniikawa-gun, Toyama 930-0397 (JP)
(74) Representative: Schiener, Jens
(86) International application number: PCT/JP2019/004789
(87) International publication number: WO 2019/159868

(57) **Abstract**

To provide a novel colitis ameliorating agent.

A colitis ameliorating agent comprising a trans-astaxanthin derivative of formula (I), geometric isomers thereof, a mixture of the geometric isomers, an optical isomer thereof, or a salt thereof; 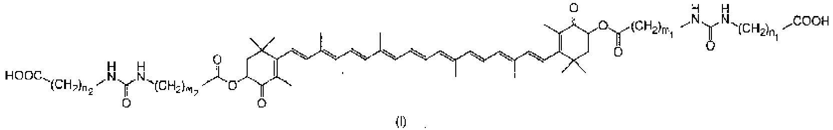 (wherein m₁, m₂, n₁ and n₂ are each the same or different and mean an integer of 1 to 6).

## Description

### TECHNICAL FIELD

The present invention relates to a colitis ameliorating agent containing an astaxanthin derivative.

### BACKGROUND

Colitis is a generic term for inflammatory diseases of the colorectal intestine.
(1) Colitis may be divided into acute and chronic colitis by the attack period. Many cases of acute colitis are infectious colitis. Many cases of chronic colitis are non-specific colitis such as ulcerative colitis and Crohn's disease.
(2) Colitis may be divided into diffused and localized colitis by the site and distribution. Typical diffused colitis includes ulcerative colitis, and typical localized colitis includes Crohn's disease and intestinal tuberculosis, respectively.
(3) In terms of infection, colitis may be divided into infectious colitis and non-infectious colitis.
   Infectious colitis is mostly due to acute colitis caused by bacterial infections, such as shigellosis, typhoid, and salmonella enteritis, whereas intestinal tuberculosis and amebiasis takes a chronic course.
(4) Colitis may be divided into specific-type colitis and nonspecific-type colitis, by etiology.
   Specific-type colitis is a generic term for colitis with distinct etiologies, including intestinal tuberculosis, shigellosis, and salmonella enteritis.
   Nonspecific-type colitis is of unknown etiology, also called idiopathic colitis, and ulcerative colitis and Crohn's disease are typical cases.
(5) Besides, there are intractable radiation colitis observed after radiotherapy of uterine cancer, colitis caused by the alternation phenomenon of bacteria by antibiotic administration, ischemic colitis complicated by arteriosclerosis, diabetes mellitus or the like, infectious colitis caused by food poisoning, and the like.

Since the causes of colitis are diverse as described above, a variety of drugs are currently selected for use in the treatment according to the causes and symptoms, and a fundamental treatment has not been established. Among them, no effective drugs were found for some colitis such as ulcerative colitis until recently, and so it has been recognized as an intractable disease which is difficult to treat. 5-aminosalicylic acid (5-ASA) formulations have been clinically available in these several years, and so it has become somewhat easier to treat colitis than before, colitis still remains a disease for which a more sufficiently effective drug is desired.

Under the circumstances, a more effective drug for treating colitis has been continuously hoped for.

Meanwhile, it is known that astaxanthin has excellent anti-oxidation activity and is known to be useful in the photolesion disease, an ophthalmic disease, a dermatologic disease, an inflammation disease, an immune disease, a cardiac disease, a malignant tumor disease, a liver disease, a kidney disease, a neurodegenerative disease, an addictive disease, an allergic disease, an insulin-resistant disease, a diabetic disease, a hyperlipidemia disease, a cardiac function disease, a vascular system disease and so on (Non-Patent Literatures 1 and 2).

As a compound retaining an anti-oxidation activity equal to or higher than that of astaxanthin and also having an improved water solubility and oral absorbability of the same compound, the compound of formula (I) below is known (Patent Literature 1): 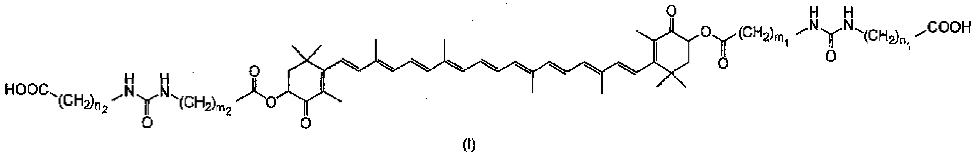 (wherein m₁, m₂, n₁ and n₂ are each the same or different integers from 1 to 6).

In Patent Literature 1, although inflammatory colitis is listed as a disease in which the compound of formula (I) can exhibit an effect, there is no disclosure of colitis or report of a specific effect on it, and further, no improvement effect of the compound on refractory colitis such as ulcerative colitis is disclosed or known.

### PRIOR ART

### PATENT LITERATURES

[Patent Literature 1]
WO 2015/178404

### [Non-Patent Literature]

[Non-Patent Literature 1]
   Alternative Medicine Review, 2000, 16(4), 355-364
[Non-Patent Literature 2]
   Trends in Biotechnology, 2003, 21(5), 210-216

### SUMMARY OF THE INVENTION

### PROBLEM TO BE SOLVED

The present invention mainly aims to provide a colitis ameliorating agent that can be substituted for the representative colitis treating agent 5-ASA and exhibits an effect comparable to or overwhelming the 5-ASA.

### SOLUTION TO PROBLEM

As a result of energetic studies to find a new therapeutic agent for ameliorating colitis, the authors have found that a trans-astaxanthin derivative of formula (I), geometric isomers thereof, a mixture of the geometric isomers, an optical isomer thereof or a salt thereof has an excellent ameliorating effect against colitis, particularly ulcerative colitis, and has completed the present invention.

That is, the present invention provides the following [1] to [4].
[1] A colitis ameliorating agent containing a trans-astaxanthin derivative of formula (I), geometric isomers thereof, a mixture of the geometric isomers, an optical isomer thereof or a salt thereof; 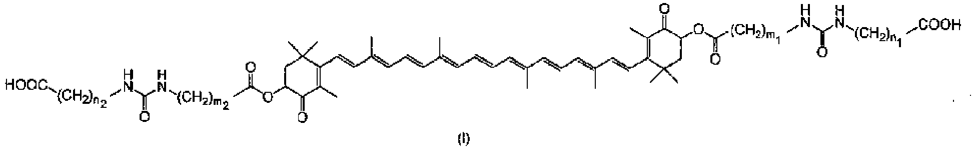 (wherein m₁, m₂, n₁ and n₂ each mean an integer of 1 to 6, which may be the same or different).
[2] Use of a trans-astaxanthin derivative of formula (I), geometric isomers thereof, a mixture of the geometric isomers, an optical isomer thereof or a salt thereof for producing a colitis ameliorating agent.
[3] A trans-astaxanthin derivative of formula (I), geometric isomers thereof, a mixture of geometric isomers thereof, an optical isomer thereof or a salt thereof for ameliorating colitis.
[4] A therapeutic method for ameliorating colitis, characterized in that an effective amount of a trans-astaxanthin derivative of formula (I), a geometric isomer thereof, a mixture of geometric isomers thereof, an optical isomer thereof or a salt thereof is administered.

### ADVANTAGEOUS EFFECT OF THE INVENTION

The trans-astaxanthin derivative of formula (I) of the present invention, a geometric isomer thereof, a mixture of the geometric isomer, their optical isomer or a salt thereof exhibit excellent effect against colitis of various animals in general such as humans, dogs, cats, horses, and the like, and a pharmaceutical composition containing an astaxanthin derivative of formula (I), a geometric isomer thereof, a mixture of geometric isomer thereof, an optical isomer thereof or a salt thereof is excellent as a colitis ameliorating agent.

As described above, colitis, which the present invention aims to improve, is classified by the duration of onset, site, distribution, cause, and the like. Among these, chronic colitis may be a disease which can be improved by the present invention, and nonspecific-type colitis such as ulcerative colitis and Crohn's disease in the colon can be cited as a disease for which it can be more expected to be effective.

### BRIEF DESCRIPTION OF THE DRAWINGS

Figure 1 is a diagram showing the transition of the weight average value in the embodiment.
Figure 2 is a diagram showing a transition of the mean value of the blood stool score in the embodiment.
Figure 3 is a diagram showing colon length of each test animal group in the examples.
Figure 4 is a diagram showing colon weight of each test animal group in the examples.

### DESCRIPTION OF EMBODIMENTS

The colitis ameliorating agent of the present invention contains a trans-astaxanthin derivative of the above formula (I), geometric isomers thereof, a mixture of the geometric isomers, an optical isomer thereof or a salt thereof as an active ingredient.

Objects that can be treated by the present invention include, but are not limited to, isothermal animals including humans; e.g., dogs, cats, horses, monkeys, rabbits, rats or mice. 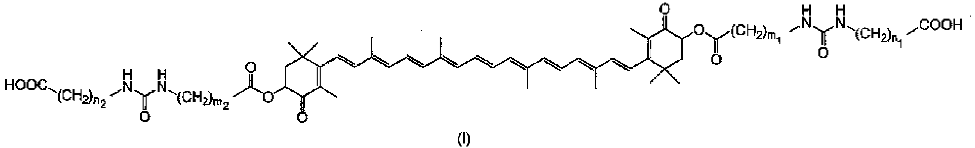 (wherein m₁, m₂, n₁ and n₂ each represent the same or different integers from 1 to 6).

Among the compound of the above formula (I), it is preferable that m₁ and m₂ are each an integer of 1, and that n₁ and n₂ are each an integer of 3.

The compound of formula (I), geometric isomers thereof, a mixture of the geometric isomers and an optical isomer thereof can form pharmaceutically acceptable salts by subjecting them to normal salt-forming reactions with base substances or base compounds desired because they have carboxyl groups within the molecule.

Such salts include, for example, sodium salts, potassium salts, alkali metal salts such as lithium salts; alkaline earth metal salts such as calcium salts, magnesium salts; amino acid salts such as lysine salts, ornithine salts, arginine salts, among which sodium salts, lysine salts may be preferable.

In the chemical structure of formula (I), the double bond moiety in the mid-chain carbon chain in the astaxanthin basic structure can assume geometric trans/cis isomers in terms of chemical structure. Regarding the active ingredient of the present invention, not only a trans-form of formula (I) but also cis-forms represented by the following formulae (Ia) and (Ib) may be included in the active ingredient of the colitis ameliorating agent of the present invention. The colitis ameliorating agent of the present invention can include a mixture of a trans-form of formula (I) and a cis-form which is a geometric isomer thereof at arbitrary ratio as active ingredient. 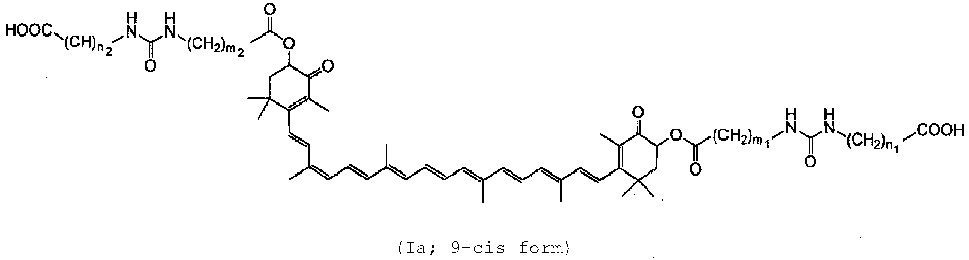 (wherein m₁, m₂, n₁ and n₂ mean the same as above). 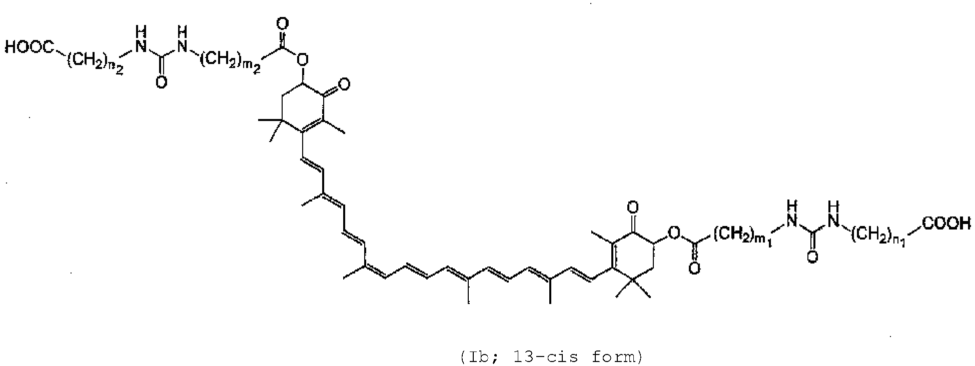 (wherein m₁, m₂, n₁ and n₂ mean the same as above) .

In addition, the compound of formula (I), geometric isomers thereof, and a mixture of the geometric isomers encompass an optical isomer of formula (IA) below, and also encompass an optical antipode thereof, a mixture thereof, and a diastereomer altogether as the active ingredient of the colitis ameliorating agent of the present invention. 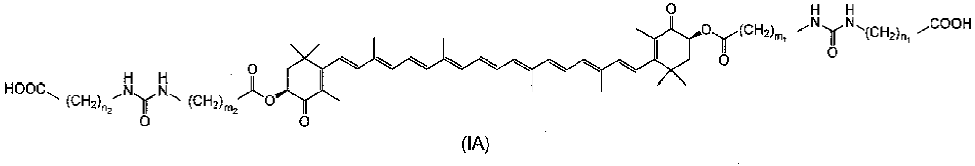 (wherein m1, m2, n1 and n2 mean the same as above).

Among the compound of the formula (I), geometric isomers thereof, a mixture of the geometric isomers, an optical isomer thereof, and a trans-form of formula (IA) is preferable.

In addition, among the trans-form of formula (IA), a compound in which m₁ and m₂ are each an integer of 1, and n₁ and n₂ are each an integer of 3 is preferable.

As described above, an optically active trans-astaxanthin derivative of formula (IA) or a salt thereof is preferable, and further an optically active trans-astaxanthin derivative of formula (IA) or a salt thereof, which is substantially free of the optically active cis-astaxanthin derivative corresponding to the optically active trans-astaxanthin derivative of formula (IA) is more preferable. Here, to contain the active ingredient of the colitis ameliorating agent of the present invention at "high purity" denotes the purity of the active ingredient is at least 95%, preferably at least 98%.

The compound of formula (I), geometric isomers thereof, an optical isomer of the geometrical isomers, and a salt thereof may be manufactured by the production method described in the specification of WO 2015/178404, or by arbitrarily combining the production method and a known method. Among the production methods, the following explains the method for manufacturing geometrical isomers of formula (I), an optical isomer thereof, by way of the production method of the optical isomer of formula (IA) as a representative.

### (1A) Deprotection reaction

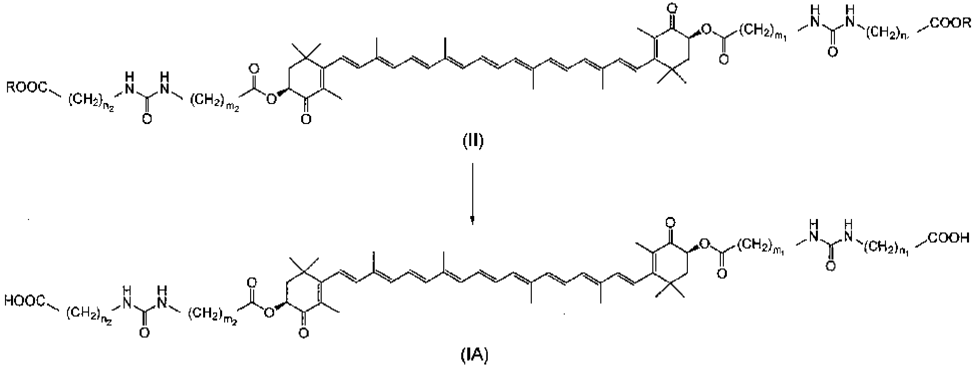

(wherein m₁, m₂, n₁, and n₂ are the same meanings as described above, and R represents a protecting group).

Deprotecting the protecting group(s) of the compound of formula (II) serving as the raw material enables to produce the compound of interest, an optically active trans-astaxanthin derivative of formula (IA).

For the deprotection reaction, normal deprotection reactions for protecting groups can be employed, e.g., deprotecting reaction by acid.

The protecting groups include a tertiary butyl, trimethylsilyl, tetrahydropyranyl, and the like, preferably tertiary butyl, trimethylsilyl, and the like.

For the acid elimination reaction, the compound of formula (II) is reacted in an inert solvent by adding an acid thereby to produce the compound (IA) of interest.

The solvent to be used is not particularly limited as long as it is inert to the present reaction and examples thereof include aliphatic hydrocarbons such as hexane, heptane, ligroin, and petroleum ether; aromatic hydrocarbons such as benzene, toluene, and xylene; halogenated hydrocarbons such as chloroform, methylene chloride, 1,2-dichloroethane, and carbon tetrachloride; nitriles such as acetonitrile and propionitrile; organic acid esters such as ethyl formate, isopropyl formate, isobutyl formate, ethyl acetate, isobutyl acetate, and butyl acetate; ethers such as diethyl ether, diisopropyl ether, tetrahydrofuran, dioxane, dimethoxyethane, diethylene glycol dimethyl ether; amides such as dimethylformamide, dimethylacetamide, and hexamethylphosphoric triamide; alcohols such as methanol, ethanol, propanol, and isopropanol; organic acids such as trifluoroacetic acid, formic acid, acetic acid, and propionic acid; water; and mixed solvents of these solvents, preferably hydrogenated hydrocarbons, nitriles, ethers, alcohols, organic acids, amides, water, and mixed solvents of these solvents, further preferably hydrogenated hydrocarbons, nitriles, alcohols, organic acids, ethers, water, and mixed solvents of these solvents, and most preferably halogenated hydrocarbons, acetonitrile, water, methanol, ethanol, isopropanol, formic acid, dioxane, tetrahydrofuran, and mixed solvents of water and these organic solvents (in the case where a protecting group is a C1-C6 alkyl group).

The acid to be used is not particularly limited as long as it is used as an acid in a typical reaction and examples include inorganic acids such as hydrochloric acid, hydrobromic acid, sulfuric acid, perchloric acid, and phosphoric acid; organic acids such as acetic acid, formic acid, oxalic acid, methanesulfonic acid, p-toluenesulfonic acid, camphorsulfonic acid, trifluoroacetic acid, and trifluoromethanesulfonic acid; Lewis acids such as zinc chloride, tin tetrachloride, boron trichloride, boron trifluoride, and boron tribromides; and acidic ion-exchange resins, preferably inorganic acids and organic acids, and most preferably hydrochloric acid, acetic acid, formic acid, and trifluoroacetic acid.

The reaction temperature varies depending on the raw material compound to be reacted, the acid and the solvent to be used, and the like and is typically from -20°C to 150°C, and preferably from 0°C to 100°C. The reaction time varies depending on the raw material compound, the solvent, the reaction temperature, and the like and is typically from 30 minutes to 10 days, and preferably from 30 minutes to 5 days. The amount of the solvent to be used is typically from 10 times to 50 times as much as the volume, and preferably 30 times as much as the volume, based on the weight of the compound of formula (II) to be used. The amount of the acid to be used is, for inorganic acids, typically from 5 to 50 times as much as the number of moles, and preferably from 10 to 30 times as much as the number of moles, and for organic acids, typically from 100 to 1,000 times as much as the number of moles, and preferably from 200 to 600 times as much as the number of moles of the raw material compound of formula (II).

The product obtained by the above deprotection reaction may contain geometric isomers such as 9-cis forms and 13-cis forms described above, and thus separation and purification means such as column chromatography, reprecipitation, and crystallization are suitably combined in accordance with the purpose to separate and remove the geometric isomers thereby to isolate and produce the optically active trans-astaxanthin derivative of formula (IA) of interest in a high purity.

Further, the separated cis-forms described above can be isolated and collected by appropriately combining purification and separation methods as described above.

### (1B) Conversion method from cis-form to trans-form

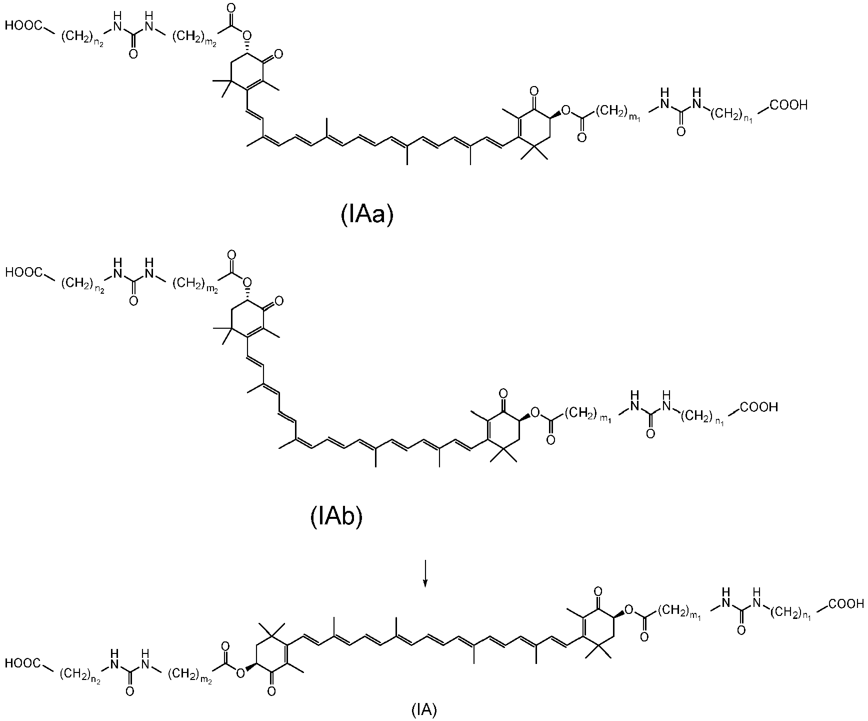

(wherein m₁, m₂, n₁ and n₂ mean the same as above) .

The representative cis-forms to be used in the present production method are the compounds of formulae (IAa) and (IAb) as described above, and these are dissolved in an inert solvent as a single raw material compound, or a mixture of cis-compounds, or a mixture with trans-forms excessively containing cis-forms and reacted using a conversion reagent such as iodine to produce the high-purity optically active trans-astaxanthin derivative of formula (IA) of interest.

The solvent to be used is not particularly limited as long as it is inert to the present reaction and examples thereof include tetrahydrofuran, ethyl acetate, acetonitrile, acetone, and water.

Examples of the above conversion reagent preferably used include iodine.

The reaction temperature varies depending on the raw material compound to be reacted, the conversion reagent and a solvent to be used, and the like and is typically from -20°C to 150°C, and preferably from 10°C to 100°C. The reaction time varies depending on the raw material compound, the solvent, the reaction temperature, and the like and is typically from 30 minutes to 10 days, and preferably from 30 minutes to 5 days. The amount of the solvent to be used is typically from 10 times to 50 times as much as the volume, and preferably 30 times as much as the volume, based on the weight of the compound of formula (IAa) or formula (IAb) to be used. The amount of the conversion reagent to be used is typically 0.01 time or more as much as the number of moles, and preferably 0.1 time or more as much as the number of moles of the raw material compound of formula (IAa) or formula (IAb).

Examples of the method for separating geometric isomers such as the above 9-cis form and 13-cis form from the product to be obtained by the above conversion reaction include methods such as column chromatography, reprecipitation, and crystallization, and a suitable combination of these methods in accordance with a purpose enables the separation of the geometric isomers and the isolation and production of the optically active trans-astaxanthin derivative of formula (IA) of interest in a high purity.

Further, the separated cis-forms can be isolated and produced as respective cis-forms when the above separation means are suitably combined and utilized.

Subsequently, a representative production method of the above raw material compound (II) is described below.

### (2A) Method for directly binding the entire side chain moiety to 3S,3'S-astaxanthin

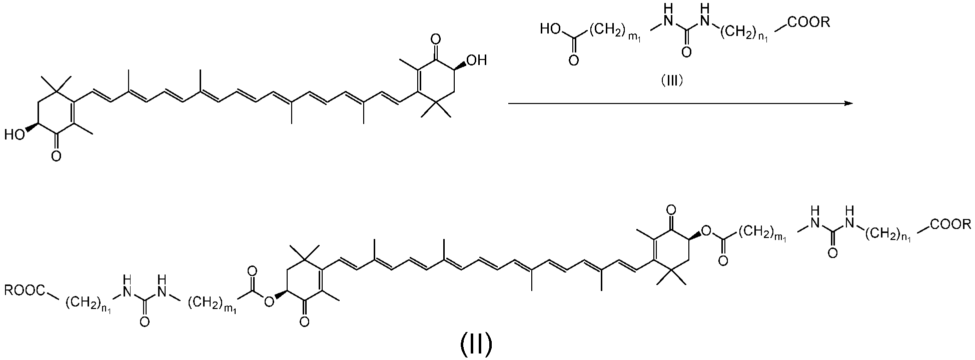

(wherein m₁ and n₁ mean the same as above, and R means a protecting group (for example, a tert-butyl group)).

3S,3'S-Astaxanthin is dissolved in an inert solvent and subsequently the compound of formula (III), which is the side chain moiety of the compound of formula (I), is reacted thereto in the presence of a condensation reagent to produce the compound of formula (II).

Examples of the solvent include organic solvents such as methylene chloride, chloroform, and carbon tetrachloride.

For the condensation reagent, those used for typical condensation reaction can be used, and specific examples include water-soluble carbodiimide hydrochlorides (for example, 1-(3-dimethylaminopropyl)-3-ethylcarbodiimide hydrochloride), N,N-diisopropylcarbodiimide, carbonyldiimidazole, and dicyclohexylcarbodiimide. The amount of a condensation reagent to be used is typically 2 times the number of moles or more, and preferably 2.5 times the number of moles to 20 times the number of moles of the raw material 3S,3'S-astaxanthin.

The compound of formula (III) which is the side chain moiety is used typically 2 times the number of moles, and preferably 2.5 times as much as the number of moles to 20 times as much as the number of moles of 3S,3'S-astaxanthin.

The reaction temperature varies depending on the raw material compound to be reacted, the condensation reagent and the solvent to be used, and the like and is typically from -20°C to 150°C, and preferably from -10°C to 100°C. The reaction time varies depending on the raw material compound, the solvent, the reaction temperature, and the like and is typically from 30 minutes to 10 days, and preferably from 30 minutes to 5 days. The amount of the solvent to be used is typically from 10 to 50 times as much as the volume, and preferably 30 times as much as the volume, based on the weight of 3S,3'S-astaxanthin to be used.

The compound of formula (II) to be obtained can be typically purified and isolated by appropriately combining purification means such as column chromatography, reprecipitation, and recrystallization.

The entire side chain moiety can be produced by the following method.

### (2A-1)

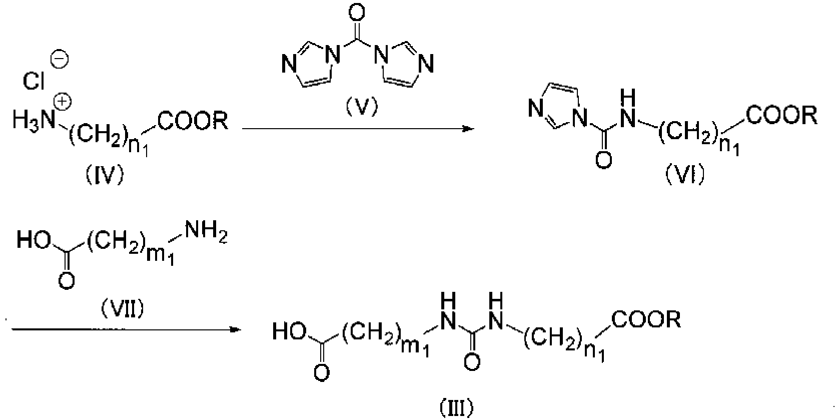

(wherein m₁ and n₁ mean the same as above, and R means a protecting group (for example, a tert-butyl group)).

Carbonyldiimidazole (V) and the compound of formula (VII) are sequentially reacted to the compound of formula (IV) to produce the compound of formula (III) of interest.

Specifically, carbonyldiimidazole (V) is reacted with the compound of formula (IV) in an inert solvent in the presence or absence of a reagent such as a base thereby to obtain the compound of formula (VI), which is an intermediate product. Further, the compound of formula (VII) is reacted to trimethylsilyl chloride in the presence of a reagent such as a base and subsequently reacted to the compound of formula (VI) thereby to produce the compound of formula (III) of interest.

Examples of the solvent used in the step of obtaining the compound of formula (VI) include organic solvents such as chloroform and methylene chloride, and the amount of these organic solvents to be used is typically from 5 times to 30 times as much as the volume, and preferably from 15 times as much as the volume, based on the weight of the compound of formula (IV) to be used.

For the basic reagent, those used for typical condensation reaction can be used, and specific examples thereof include triethylamine, N,N-diisopropylethylamine, pyridine, and N,N-dimethylaminopyridine.

The reaction temperature varies depending on the raw material compound to be reacted, the reagent and the solvent to be used, and the like and is typically from - 20°C to 150°C, and preferably from 0°C to 30°C. The reaction time varies depending on the raw material compound, the solvent, a reaction temperature, and the like and is typically from 15 minutes to 10 days, and preferably from 30 minutes to 2 days.

Examples of the solvent for reacting trimethylsilyl chloride and the compound of formula (VII) in the step of obtaining the compound of formula (III) of interest include organic solvents such as chloroform, methylene chloride, and pyridine, and the amount of these solvents to be used is typically from 5 times to 50 times as much as the volume, and preferably 20 times as much as the volume, based on the weight of the compound of formula (VII) to be used.

For the base, those used for typical condensation reaction can be used, and specific examples thereof include triethylamine, N,N-diisopropylethylamine, pyridine, and N,N-dimethylaminopyridine. The amount of a base and a reagent to be used is typically 2 times or more as much as the number of moles, and preferably 2.5 times as much as the number of moles to 5.0 times as much as the number of moles of the raw material compound of formula (VI).

The reaction temperature varies depending on the raw material compound to be reacted, the reagent and the solvent to be used, and the like and is typically from - 20°C to 100°C, and preferably from 0°C to 30°C. The reaction time varies depending on the raw material compound, the solvent, the reaction temperature, and the like and is typically from 15 minutes to 5 days, and preferably from 30 minutes to 2 days. The reaction temperature when subsequently the compound of formula (VI) is added and reacted varies depending on the raw material compound to be reacted, the reagent and the solvent to be used, and the like and is typically from - 20°C to 150°C, and preferably from 10°C to 60°C. The reaction time varies depending on the raw material compound, the solvent, the reaction temperature, and the like and is typically from 30 minutes to 10 days, and preferably from 30 minutes to 4 days.

### (2B) Method for sequentially binding side chain moiety parts to 3S,3'S-astaxanthin

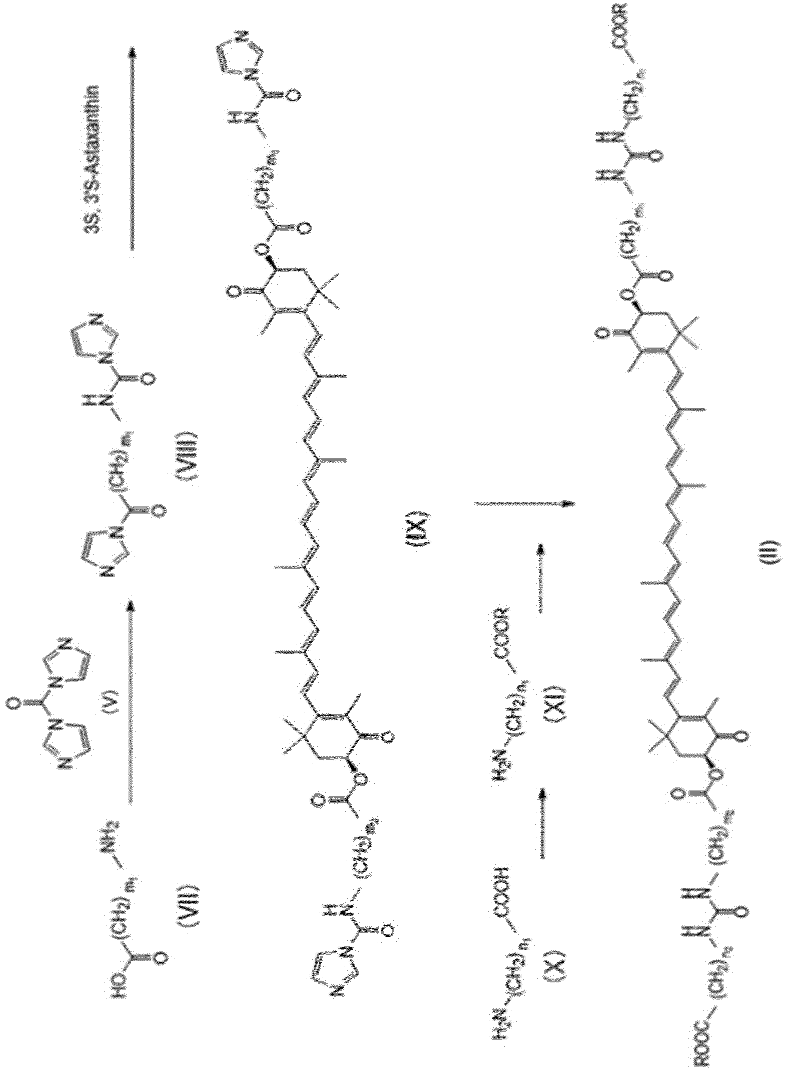

(wherein m₁, m₂, n₁ and n₂ mean the same as above, and R means a protecting group (for example, a tert-butyl group or a trimethylsilyl group)).

The present production method can be performed basically by binding the side chain moiety part (VIII) obtained by reacting the compound of formula (VII) and carbonyldiimidazole (V) to 3S,3'S-astaxanthin, and subsequently binding the side chain moiety part (XI) to the obtained product (IX).

Each of the reaction conditions shown in the above production method (2A-1) may be similarly used in the step in which carbonyldiimidazole (V) is used.

Examples of the solvent include organic solvents such as chloroform and methylene chloride, and the amount of these organic solvents to be used may be typically from 2 times to 30 times as much as the volume, and preferably 7 times as much as the volume, based on the weight of the compound of formula (VII) to be used.

The reaction temperature varies depending on the raw material compound to be reacted, the reagent and the solvent to be used, and the like and is typically from - 20°C to 150°C, and preferably from -10°C to 100°C. The reaction time varies depending on the raw material compound, the solvent, the reaction temperature, and the like and is typically from 30 minutes to 10 days, and preferably from 30 minutes to 5 days. Examples of the base include triethylamine, N,N-diisopropylethylamine, pyridine, and N,N-dimethylaminopyridine.

For the binding reaction of the side chain moiety part (VIII) to be obtained and 3S,3'S-astaxanthin, the reactions are proceeded similarly to the reaction 2A described above thereby to produce the compound of formula (IX).

The step of obtaining the formula (II) of interest can be performed by reacting the formula (XI) to the compound having the formula (IX) obtained above. The present reaction is carried out in accordance with the method for producing the above formula (VIII).

The reaction temperature varies depending on the raw material compound to be reacted, the reagent and the solvent to be used, and the like and is typically from - 20°C to 100°C, and preferably from 0°C to 40°C. The reaction time varies depending on the raw material compound, the solvent, the reaction temperature, and the like and is typically from 30 minutes to 10 days, and preferably from 30 minutes to 30 hours.

Regarding the method for producing the compound of formula (XI), (1) when R is a t-butyl group, it can be performed in accordance with a generally known method for synthesizing t-butyl ester of an amino acid or (2) when R is a trimethylsilyl group, it can be performed by reacting trimethylsilyl chloride to the compound having the formula (X) in an inert solvent in the presence of a base (it can be performed in accordance with the method for producing the compound of the above formula (III)). The reaction (2) can be performed in accordance with a generally known method for silylating a hydroxyl group and a carboxyl group. When R in the formula (XI) is a trimethylsilyl group, the trimethylsilyl group can be easily eliminated by using water or mildly acidic water for after-treatment of the reaction for producing the formula (II).

Suitable combination of typical purification means such as column chromatography, reprecipitation, and recrystallization used on the obtained product enables the production of the compound of formula (II) of interest.

The compound of formula (I) of the present invention, geometric isomers thereof, a mixture of the geometric isomers, an optical isomer thereof, or a salt thereof can be administered as an oral formulation , an injection, a suppository, or an enema formulation by which a drug is directly injected from the anus.

For the production of solution formulations such as a drink formulation, an injection, and an enema formulation, an active ingredient is used by the typical formulation technology in the presence of a pH adjusting agent, a buffer, a dissolving agent, a suspending agent, a tonicity agent, a stabilizer, or a preservative as needed. Examples of the pH adjusting agent include hydrochloric acid, sodium hydroxide, potassium hydroxide, and triethanolamine. Examples of the buffer include sodium phosphate, sodium acetate, sodium borate, sodium citrate, and sodium aspartate. Examples of the suspending agent include polysorbate 80, methyl cellulose, hydroxyethyl cellulose, sodium carboxymethyl cellulose, polyoxyethylene sorbitan monolaurate, gum arabic, tragacanth powder, polyvinylpyrrolidone, and glyceryl monostearate. Examples of the dissolving agent include polysorbate 80, polyoxyethylene hydrogenated castor oil, nicotinic acid amide, polyoxyethylene sorbitan monolaurate, macrogol, castor oil fatty acid ethyl ester, Vaseline, glycerin, and propylene glycol. Examples of the stabilizer include sodium sulfite, sodium metasulfite, sodium citrate, sodium edetate, and monoethanolamine. Examples of the preservative include methyl p-hydroxybenzoate, ethyl p-hydroxybenzoate, sodium benzoate, sorbic acid, phenol, cresol, chlorocresol, benzalkonium chloride, and paraben.

A solid oral formulation is administered in any form such as a tablet, a capsule, a granule, or a powder, and can be produced by suitably mixing an active ingredient with pharmaceutically acceptable medical additives such as an excipient, a disintegrator, and a binder using a typical formulation technology.

A suppository can be produced by combining an active ingredient with a widely used suppository base such as vaseline using a typical formulation technology.

When the compound of formula (I) of the present invention, geometric isomers thereof, a mixture of the geometric isomers, an optical isomer thereof, or a salt thereof is administered, administration method and administration preparation are appropriately selected depending on the symptoms, patient's age, body weight, and sex, or general health conditions. For example, the administration, when made to a warm-blooded animal having a body weight of about 70 kg orally or from the anus, is carried out in a daily dose of from 0.01 to 1,000 mg, preferably from 1 to 100 mg, and more preferably from 5 to 20 mg once daily or more, for example, from 1 to 6 times. Further, the administration, when given as an injection, is typically carried out to an adult intravenously in a daily dose of from 5.0 to 80.0 mg with a suitably increased or decreased dose depending on the symptoms.

The compound of formula (I) of the present invention, geometric isomers thereof, a mixture of the geometric isomers, an optical isomer thereof, or a salt thereof have no particular safety issue within the above dosage ranges.

### EXAMPLES

Hereinafter, the present invention will be described in reference to Examples. However, the scope of the present invention is not limited in any way to the following Examples.

### Example 1

In this Example, mouse models to which dextran sulfate sodium salt (DSS) is used will be described as colitis models. When DSS is administered by being mixed in drinking water of the mouse, an experimental ulcerative colitis model similar to human ulcerative colitis in terms of symptoms such as suppression of body weight increase, blood in stools, and anemia and formation of colon erosion and further lacking intestinal lesion was build (Non-Patent Literature 3). A suppression effect of Compound X on colitis in such a model as the representative colitis model was examined. For the evaluation items, recovery from diarrhea and body weight loss by colitis, and the length and weight of the colon were examined as changes in colon properties, which are widely known to occur by DSS-induced colitis.

In this Example, the following compound was used as the representative compound of formula (I). Hereinafter, in this Example, this compound is described as Compound X.

Compound X: 4-(3-14-[18-(4(S)-[3-(3-Carboxypropyl)ureidoacetoxy]-2,6,6-trimethyl-3-oxocyclohexa-1-enyl)-3,7,12,16-tetramethyloctadeca-1(E), 3(E), 5(E), 7(E), 9(E), 11(E), 13(E), 15(E), 17(E)-nonaenyl]-3,5,5-trimethyl-2-oxocyclohex-3-enyl-1(S)-oxycarbonylmethyl}ureido)butyric acid dilysine salt

### (1) Producing pathological model animal

For treatment, C57BL/6J, male mice were purchased from CHARLES RIVER LABORATORIES JAPAN, INC. in accordance with previous literature (Non-Patent Literature 2) and 5-week old mice with no abnormal general conditions during quarantine and acclimation period were used for the experiment. After acclimation, the animals were divided in such a way as to be 1 mouse per cage. DSS used in the experiment having a molecular weight of from 36,000 to 50,000 Da was purchased from MP Bio Japan. DSS was added to tap water in such a way as to be 3% and suspended for causing colitis. The suspension was prepared once every 3 days and freely provided to each of the mice as water supply. A solution from which DSS was removed was provided as water supply to a non-pathological control group.

### (2) Treatment

After 5-day DSS treatment, symptoms of blood in stools and diarrhea were confirmed to score the pathological conditions and evenly divided into each group as shown in the following Table 1. 5-Aminosalicylic acid (hereinafter 5-ASA, Tokyo Chemical Industry, Co., Ltd.) was used in accordance with Non-Patent Literature 2 as a positive control of the test. After group division, each of the test substances of Table 1 was suspended in a solution of 0.5% sodium carboxymethyl cellulose (hereinafter, CMC, Nacalai Tesque Inc.) in such a way as to be each treatment of Table 1 and orally administered once daily. A group to which DSS treatment was not carried out and further both of the test substances Compound X and 5-ASA were not administered was set to be a control group, Sham group, and a group to which only DSS treatment was carried out but both of the test substances Compound X and 5-ASA were not administered was set to be DSS group. These control groups, Groups 1 and 2, were orally administered, as the test substance, with the 0.5% CMC solution used as the medium.

Drinking water after the group division was typical water for drinking.

**[Table 1]**

| Experimental treatment group | | | | |
|---|---|---|---|---|
| No. | Test group | DSS Treatment | Test substance | Test substance administration route |
| 1 | Sham | Tap water | CMC | Oral administration |
| 2 | DSS + Vehicle | 3%DSS | CMC | |
| 3 | DSS + Compound X 1 mg/kg/day group | 3%DSS | Compound X | |
| 4 | DSS + Compound X 10 mg/kg/day group | 3%DSS | Compound X | |
| 5 | DSS + Compound X 100 mg/kg/day group | 3%DSS | Compound X | |
| 6 | DSS+5-ASA 50 mg/kg/day group | 3%DSS | 5-ASA | |

**[Table 2]**

| Fecal scoring criteria | |
|---|---|
| Score | Condition |
| 0 | Normal stools |
| 1 | Blood in stools |
| 2 | Blood adhered to the anus |
| 3 | Gross bleeding |

### (3) Measurement and Results

The test started with 6 mice in Group No. 1 and 10 mice each in Group Nos. 2 to 6. After the treatment started, death of animals in Group Nos. 2 to 6 due to the cause considered enteritis from DSS was observed and thus 6 mice in Group Nos. 1 and 2 and 5 mice in Group Nos. 3 to 6 were finally used for analysis.

### (3. 1) Measurement of body weight

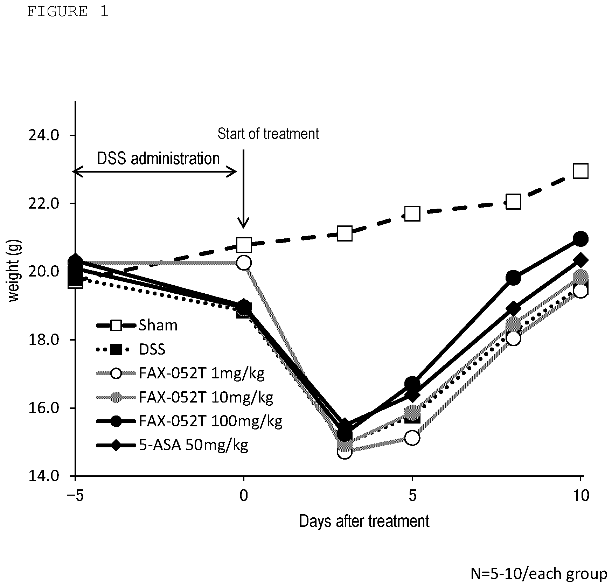

Body weights were measured on the DSS administration starting day (day -5), DSS administration last day (day 0), on day 3, day 5, day 8, and day 10 of test substance administration. The results are shown below in Table 3 and Figure 1. As shown in Figure 1, Sham group of Group No. 1 to which DSS administration was not carried out was found to have had gradual body weight increases, whereas all groups to which DSS treatment was carried out were found to have had body weight losses up to day 3 of the test substance administration. Thereafter, all groups were found to have had body weight increases due to the recovery of pathological conditions. The 5-ASA administration group of Group No. 6, the positive control group, had significant body weight recovery compared with the DSS administration only, and body weight recovery was even further significant in 100 mg/kg/day Compound X administration group. Thus, it was suggested that Compound X has potential to exhibit a therapeutic effect on colitis caused by DSS.

**[Table 3]**

| Body weight changes (Unit: g, average value ± standard deviation) | | | | | | | |
|---|---|---|---|---|---|---|---|
| No. | Test group | day-5 | day 0 | day 3 | day 5 | day 8 | day 10 |
| 1 | Sham | 19.7±0.8 | 20.8±1.3 | 21.1±1.4 | 21.7±2.1 | 22.1±1.9 | 23.0±2.0 |
| 2 | DSS | 19.8±0.8 | 18.9±1.3 | 15.0±1.4 | 15.8±2.1 | 18.3±1.9 | 19.6±2.0 |
| 3 | DSS + Compound X 1 mg/kg | 20.3±0.9 | 20.3±0.8 | 14.7±1.3 | 15.1±1.5 | 18.0±2.4 | 19.4±2.2 |
| 4 | DSS + Compound X 10 mg/kg | 20.1±0.9 | 18.9±1.1 | 14.9±1.4 | 15.9±1.9 | 18.5±2.1 | 19.8±1.7 |
| 5 | DSS + Compound X 100 mg/kg | 20.1±0.9 | 19.0±1.0 | 15.2±1.8 | 16.7±2.2 | 19.8±0.9 | 21.0±0.7 |
| 6 | DSS+5-ASA 50 mg/kg | 20.3±0.9 | 19.0±1.0 | 15.5±1.0 | 16.4±1.7 | 18.9±1.2 | 20.3±1.5 |

### (3.2) Fecal scoring

The therapeutic effect was evaluated by observing changes in blood in stools caused by enteritis as the body weight recovery alone can be considered that the body weight increase might have been promoted simply by the growth. Mice feces were observed on the DSS administration starting day (day-5), DSS administration last day (day 0), on day 3, day 5, day 8, and day 10 of test substance administration and scored in accordance with the criteria of Table 2.

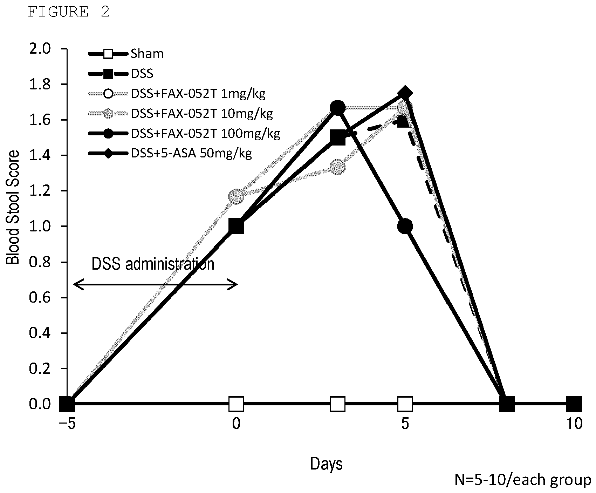

The results are shown in Table 4 and Figure 2 below. Sham group to which DSS administration was not carried out had only normal feces and no abnormality was found, whereas all groups to which DSS treatment was carried out had aggravated blood feces scores from days 3 to 5 of test substance administration. However, quick recovery was found thereafter as in the body weight recovery. The positive control group 5-ASA did not have a difference between time-dependent changes in blood feces score from those in the DSS administration group, whereas the 100 mg/kg/day Compound X administration group was found to exhibit improved blood feces score on day 5 of test substance administration. Thus, it was also found from the observation data of blood feces that Compound X exhibits a therapeutic action on colitis caused by DSS.

**[Table 4]**

| Fecal scoring average value changes (average value + standard deviation) | | | | | | | |
|---|---|---|---|---|---|---|---|
| No. | Test group | day -5 | day 0 | day 3 | day 5 | day 8 | day 10 |
| 1 | Sham | 0.0±0.0 | 0.0±0.0 | 0.0±0.0 | 0.0±0.0 | 0.0±0.0 | 0.0±0.0 |
| 2 | DSS | 0.0±0.0 | 1.0±0.0 | 1.5±0.5 | 1.6±0.5 | 0.0±0.0 | 0.0±0.0 |
| 3 | DSS + Compound X 1 mg/kg | 0.0±0.0 | 1.2±0.4 | 1.7±0.8 | 1.7±0.6 | 0.0±0.0 | 0.0±0.0 |
| 4 | DSS + Compound X 10 mg/kg | 0.0±0.0 | 1.2±0.4 | 1.3±0.5 | 1.7±0.5 | 0.0±0.0 | 0.0±0.0 |
| 5 | DSS + Compound X 100 mg/kg | 0.0±0.0 | 1.0±0.0 | 1.7±0.8 | 1.0±0.0 | 0.0±0.0 | 0.0±0.0 |
| 6 | DSS+5-ASA 50 mg/kg | 0.0±0.0 | 1.0±0.0 | 1.5±0.5 | 1.8+1.0 | 0.0±0.0 | 0.0±0.0 |

### (3.3) Colon length and weight

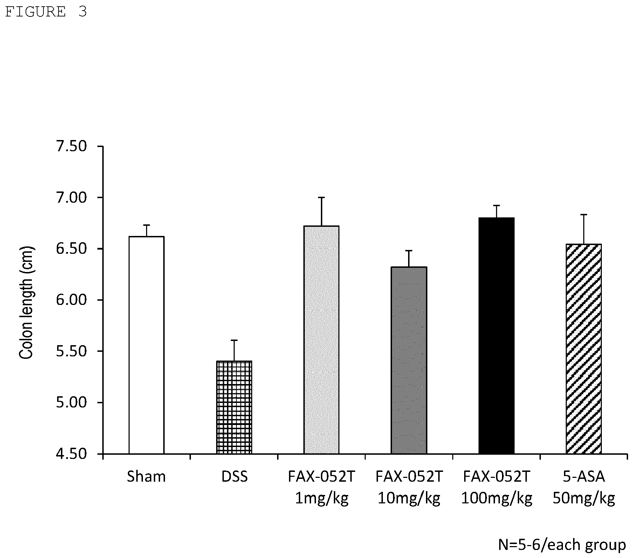

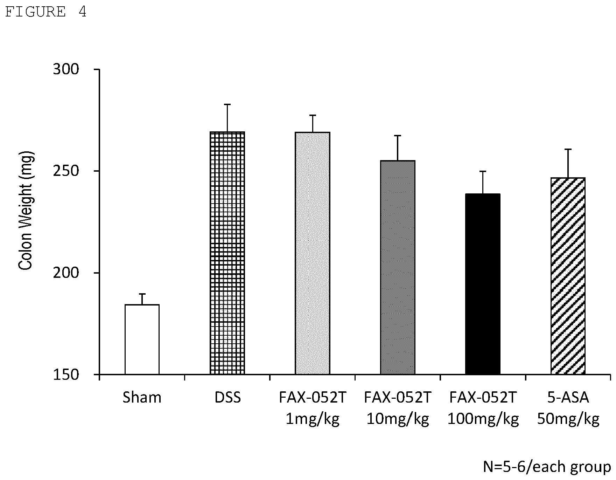

It is generally acknowledged as a known fact that DSS treatment causes colon tissue changes such as the length of colon is shortened while the weight increases. For evaluating the therapeutic effect of Compound X, the length and weight of the colon were measured. The mice were euthanized under anesthesia on day 10 of test substance administration and dissected to remove the colon. The removed colon was quickly measured for a length in a relaxed state. Subsequently, feces in the intestinal tract were removed and sufficiently washed with phosphate buffered saline (Wako Pure Chemical Industries, Ltd.) and measured for a wet weight. The lengths of colons were shown in Table 5 and Figure 3, and the wet weights of colons were shown in Table 5 and Figure 4.

**[Table 5]**

| Colon length and wet weight in DSS model mice (average value ± standard deviation) | | | |
|---|---|---|---|
| No. | Test group | Length (cm) | Wet weight (g) |
| 1 | Sham | 6.62±0.11 | 184.3±5.3 |
| 2 | DSS | 5.40±0.21 | 269.2±13.5 |
| 3 | DSS + Compound X 1 mg/kg | 6.72±0.28 | 269.0±8.4 |
| 4 | DSS + Compound X 10 mg/kg | 6.32±0.16 | 255.0±12.4 |
| 5 | DSS + Compound X 100 mg/kg | 6.80±0.12 | 238.6±11.3 |
| 6 | DSS+5-ASA 50 mg/kg | 6.54±0.29 | 246.6±14.1 |

Measurement of the colon length revealed that DSS group of Group No. 2 (5.40 cm) was short compared with Sham group of Group No. 1 (average 6.62 cm). It was revealed that Compound X groups of Group Nos. 3 to 5 and 5-ASA group of Group No. 6 had longer length of the colon than DSS group as shown in Table 5 and Figure 3. 1 and 100 mg/kg/day Compound X administration groups of Group Nos. 3 to 5 had average value of the colon length of 6.72 cm and 6.80 cm respectively, and 5-ASA had 6.54 cm. Thus, it was revealed that Compound X had an effect equivalent to or more than 5-ASA in terms of the colon length. DSS group (269.2 mg) had a heavier weight compared with Sham group (average 184.3 mg) in terms of the colon wet weight. Compound X groups and 5-ASA group had suppressed weight increases compared with DSS group as shown in Table 5 and Figure 4. Compound X 100 mg/kg/day administration group and 5-ASA group had 238.6 and 246.6 mg respectively, and it was revealed that Compound X has an effect equivalent to or more than 5-ASA. The above findings revealed that Compound X shows suppression action on morphological changes caused by DSS colitis equivalent to or more than 5-ASA.

### (4) Conclusion

The above results showed that Compound X showed the ameliorating action at least equivalent to 5-ASA on various symptoms of DSS-induced colitis thereby making it obvious to have the therapeutic action on DSS-induced colitis. Further, the Compound-X high concentration group (100 mg/kg/day administration group) had overwhelming improvement in body weight recovery, blood feces score, colon length, and colon weight over the 5-ASA administration, thereby revealing that Compound X shows better therapeutic effect on DSS-induced colitis than 5-ASA, which is an existing therapeutic drug.

### (Non-Patent Literature 3-1)

Kimura Isami, Nagahama Shinobu, Kawasaki Maki, Kamiya Akemi, Kataoka Mikiko, Study on the experimental ulcerative colitis model induced by dextran sulfate sodium in rats (the second report) - Consideration on evaluation method of drug therapeutic effects -, Folia Pharmacologica Japonica Nippon Yakurigaku Zasshi, vol. 105 (1995) No. 3, p145-152

### (Non-Patent Literature 3-2)

Moul DeyEmail author, Peter Kuhn, David Ribnicky, VummidiGiridhar Premkumar, Kenneth Reuhl and Ilya Raskin, Dietary phenethylisothiocyanate attenuates bowel inflammation in mice, BMC Chemical Biology 201010:4

### Example 2

In this Example, mouse models to which oxazolone (4-ethoxymethylene-2-phenyl-2-oxazolin-5-on, hereinafter, Oxa) is used will be described as colitis models. When Oxa was first applied to skin, subsequently administered enterally after a certain period of time, an experimental ulcerative colitis model similar to human ulcerative colitis in terms of not only symptoms such as suppression of body weight increase, blood in stools, and anemia and formation of colon erosion but also immune cells was built(Non-Patent Literature 4). A suppression effect of Compound X on colitis in such a model as the test model showing pathological conditions more similar to human ulcerative colitis was examined. For the examination items, the length and weight of the colon were examined as changes in colon properties are widely known to occur also in this model.

### (1) Producing pathological model animal

For treatment, Balb/c, male mice were purchased from CHARLES RIVER LABORATORIES JAPAN, INC. in accordance with previous literature (Non-Patent Literature 5) and 8-week old mice with no abnormal general conditions during quarantine and acclimation period were used for the experiment. After acclimation, the animals were divided in such a way as to be 1 mouse per cage. Oxa used in the experiment was purchased from Sigma.

Fur at back of the neck was shaved in an area of 1.5 x 1.5 cm under anesthesia, and 150 µL (a 100% ethanol solution) of 3% (wt/vol) Oxa was added using a pipette and applied between the shoulders to sensitize (Day 1). The mice were kept under such a typical breeding environment, fasted for 16 hours from Day 7 (water supply was continued), and subsequently on Day 8 100 µL of a solution of 1% (wt/vol) Oxa in 50% ethanol was transanally administered into the rectum (3.5 cm from the anus) using a catheter under anesthesia. Hereinafter, the above treatment is described as Oxa treatment. After treatment, typical feeding breeding was carried out.

### (2) Treatment

Starting on the day following intrarectal administration of Oxa, test substances were orally administered (once daily, for 6 days). 5-Aminosalicylic acid (hereinafter 5-ASA, Tokyo Chemical Industry, Co., Ltd.) and prednisolone (hereinafter, PSL, Sigma), which are anti-inflammatory agents used for treating ulcerative colitis, were used as positive controls for the test. After group division, each of the test substances of Table 6 was suspended in a solution of 0.5% sodium carboxymethyl cellulose (hereinafter, CMC, Nacalai Tesque Inc.) in such a way as to be each treatment of Table 6 and orally administered once daily. A group to which Oxa treatment was not carried out and further Compound X and the test substances to be positive controls were not administered was set to be a control group, Sham group (Group No. 1), and a group to which only Oxa treatment was carried out and both of the test substances Compound X and 5-ASA were not administered was set to be a Vehicle group (Group No. 2). These control groups, Groups 1 and 2, were orally administered, as the test substance, with the 0.5% CMC solution which was used as the medium.

**[Table 6]**

| group | Test group | Administration | Administration route | Oxa Treatment |
|---|---|---|---|---|
| 1 | Sham | 0.5% CMC solution | Oral | No |
| 2 | Vehicle | 0.5% CMC solution | Oral | Yes |
| 3 | Compound X low dose | Compound X, 3 mg/kg/day | Oral | Yes |
| 4 | Compound X high dose | Compound X, 10 mg/kg/day | Oral | Yes |
| 5 | 5-ASA | 5-ASA, 50 mg/kg/day | Oral | Yes |
| 6 | PSL | PSL, 5 mg/kg/day | Oral | Yes |

### (3) Measurement and results

The test started with 6 mice in Group No. 1 and 9 mice each in Group Nos. 2 to 6. After the treatment started, death of animals in Group Nos. 2 to 6 due to the cause considered enteritis from Oxa treatment was observed and thus 6 mice in Group Nos. 1 and 3, and 5 mice in Group Nos. 2 and 6, and 7 mice in Group Nos. 4 and 5 were finally used for analysis.

### (3.1) Colon length and weight

It is generally acknowledged as a known fact that Oxa treatment causes colon tissue changes such as the length of colon is shortened while the weight increases. For evaluating the therapeutic effect of Compound X, the length and weight of the colon were measured. The mice were euthanized under anesthesia on day 7 of Oxa treatment and dissected to remove the colon. The removed colon was quickly measured for a length in a relaxed state. Subsequently, feces in the intestinal tract were removed and sufficiently washed with phosphate buffered saline (Wako Pure Chemical Industries, Ltd.) and measured for a wet weight. The lengths of colons and the wet weights of colons per unit length were shown in Table 7.

**[Table 7]**

| Colon length and wet weight per length in Oxa model mice (average value ± standard deviation) | | | |
|---|---|---|---|
| group | Test group | Colon length (cm) | Colon weight (mg/mm) |
| 1 | Sham | 9.00 ± 0.25 | 2.31 ± 0.23 |
| 2 | Vehicle | 7.18 ± 0.35 | 4.21 ± 0.55 |
| 3 | Compound X low dose | 8.72 ± 0.50 | 3.44 ± 0.45 |
| 4 | Compound X high dose | 8.74 ± 0.48 | 2.80 ± 0.43 |
| 5 | 5-ASA | 7.41 ± 0.37 | 4.12 ± 0.56 |
| 6 | PSL | 8.20 ± 0.52 | 3.37 ± 0.36 |

Measurement of the colon length revealed that Vehicle group of Group No. 2 (7.18 cm) was short compared with Sham group of Group No. 1 (average 9.00 cm), thereby observing morphological changes of the colon caused by Oxa treatment. It was revealed that Compound X groups of Group Nos. 3 and 4 and 5-ASA group of Group No. 5 and PSL group of Group No. 6 had longer length of the colon than Vehicle group as shown in Table 7. Compound X 3 mg/kg/day and 10 mg/kg/day administration groups of Group Nos. 3 and 4 had average value of the colon length of 8.72 cm, 8.74 cm respectively, 5-ASA had 7.41 cm, and PSL had 8.20 cm. Thus, it was revealed that Compound X had an effect more than 5-ASA and equivalent to or more than PSL in terms of the colon length. Similarly, Vehicle group (4.21 mg) had a heavier weight compared with Sham group (average 2.31 mg) in terms of the colon wet weight. Compound X groups of Group No. 3 and 4 and 5-ASA group of Group No. 5 and PSL group of Group No. 6 had suppressed weight increases compared with Vehicle group as shown in Table 7. Compound X 3 mg/kg/day administration group and 10 mg/kg/day administration group and 5-ASA group and PSL treatment group had 3.44, 2.80, 4.12, and 3.37 mg/mm respectively, and it was revealed that Compound X has an effect more than 5-ASA and equivalent to or more than PSL. The above findings revealed that Compound X shows suppression action on morphological changes of the colon in colitis caused by Oxa treatment equivalent to or more than 5-ASA and PSL.

### (4) Conclusion

The above results showed that Compound X showed ameliorating action at least equivalent to or more than 5-ASA and PSL on various symptoms of Oxa-induced colitis thereby making it self-evident to exhibit a therapeutic action on Oxa-induced colitis. Further, the Compound X of each concentration group (3 and 10 mg/kg/day administration groups) had overwhelming amelioration in colon length and colon weight over the 5-ASA administration and equivalent to or more than PSL which is a steroid, thereby revealing that Compound X shows better therapeutic effect on Oxa-induced colitis than 5-ASA and PSL, which are existing therapeutic drugs.

### (Non-Patent Literature 4)

Heller F, Fuss IJ, Nieuwenhuis EE, Blumberg RS, Strober W., Oxazolone colitis, a Th2 colitis model resembling ulcerative colitis, is mediated by IL-13-producing NK-T cells., Immunity.17(5):629-38 (2002)

### (Non-Patent Literature 5)

Wirtz S, Popp V, Kindermann M, Gerlach K, Weigmann B, Fichtner-Feigl S, Neurath MF., Chemically induced mouse models of acute and chronic intestinal inflammation., Nat Protoc. 12(7):1295-1309(2017).

### (Production Example of Compound X)

Geometric isomers in the astaxanthin backbone mid-chain carbon chain moiety in the formula hereafter are conveniently indicated by the formula of the all-trans form.

### (3.1) Synthesis of t-butyl 4-(imidazol-1-ylcarbonylamino)butyrate (wherein t means tertiary; hereinafter the same):

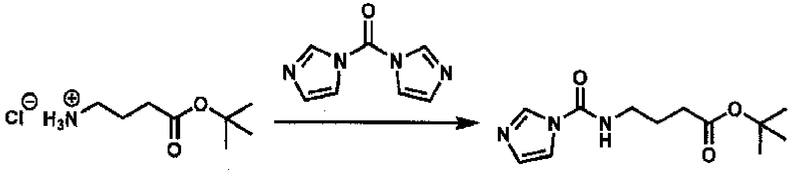

To carbonyldiimidazole (9.95 kg) was added methylene chloride (79.6 kg) and the mixture was stirred. Thereto was added a solution of 4-aminobutyric acid t-butyl hydrochloride (8.0 kg) in methylene chloride (53.1 kg) at -5 to 5°C, and the reaction mixture was stirred at the same temperature for 30 minutes. The reaction mixture was warmed to 15 to 25°C and then stirred at the same temperature for 1 hour. To the reaction mixture was added water (40 kg), and the mixture was stirred, and then the organic layer was separated. The resulting solution was washed with 5% sodium chloride aqueous solution (42.1 kg), dried over anhydrous magnesium sulfate and concentrated under a reduced pressure to give the title crude product (concentrated residue, 10.4 kg).
NMR spectrum (δ ppm, CDCl₃): 8.22 (1H, s), 7.92 (1H, br), 7.28 (1H, d, J=0.8 Hz), 7.05 (1H, d, J=0.8 Hz), 3.45 (2H, dt, J=6.0, 6.0 Hz), 2.40 (2H, t, J=6.4), 1.92 (2H, tt, J=6.4, 6.4 Hz), 1.44 (9H, s) .
Mass spectrum (+ESI, m/z): 254.00(M+H)⁺.

### (3.2) Synthesis of t-butyl 4-(3-carboxymethylureide) butyrate:

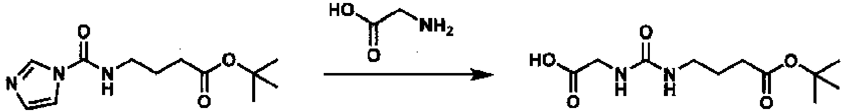

To a compound of t-butyl 4-(imidazol-1-ylcarbonylamino)butyrate (compound (3.1) above, 10.4 kg) were added methylene chloride (185.7 kg), glycine (7.4 kg), and triethylamine (8.3 kg), and the mixture was stirred. To the mixture was added chlorotrimethylsilane (8.9 kg) at -5 to 5°C, and the reaction mixture was stirred at 15 to 30°C for 60 hours. The reaction mixture was concentrated under a reduced pressure, a mixture of ethyl acetate (208 kg), hydrochloric acid (5.66 kg) and 20% sodium chloride aqueous solution (106 kg) was added thereto, and the mixture was stirred, and then the organic layer was separated. The resulting solution was washed with a mixture of hydrochloric acid (5.66 kg) and 20% sodium chloride aqueous solution (106 kg). The aqueous layer was extracted with ethyl acetate (57.4 kg), and the organic layer and the extract solution were combined. The resulting solution was washed with 20% sodium chloride aqueous solution (100 kg), dried over anhydrous magnesium sulfate and concentrated under a reduced pressure. Ethyl acetate (14.4 kg) was added and the mixture was stirred to make a homogeneous solution at 45 to 55°C. The solution was cooled to 20 to 30°C, thereto was added n-heptane (108.7kg) dropwise. After the precipitation of crystals was confirmed, the solution was stirred for 1 hour. The precipitated crystals were collected by filtration to obtain the title compound (7.98 kg, purity 99.2%) as white crystals.

The purity of the product was determined by using high performance liquid chromatography (column: YMC-Triart C18 ExRS manufactured by YMC Co., Ltd., mobile phase: phosphate buffer of acetonitrile/pH 8 = 3/7, flow rate: 1 mL/min, detection wavelength: 210 nm)
NMR spectrum (δ ppm, CDCl₃): 6.16 (1H, t, J=5.6 Hz), 6.01 (1H, t, J=5.6 Hz), 3.67 (2H, d, J=6.0 Hz), 2.97 (2H, dt, J=6.4, 6.4 Hz), 2.17 (2H, t, J=7.2 Hz), 1.56 (2H, tt, J=7.2, 7.2 Hz), 1.39 (9H, s).
Mass spectrum (+ESI, m/z): 260.92 (M+H)⁺.

### (3.3) Synthesis of t-butyl 4-(3-{4-[18-(4(S)-[3-(3-t-butoxycarbonylpropyl)ureidoacetoxy]-2,6,6-trimethyl-3-oxocyclohexa-1-enyl)-3,7,12,16-tetramethyloctadeca-1(E), 3(E), 5(E), 7(E), 9(E), 11(E), 13(E), 15(E), 17(E)-nonaenyl]-3,5,5-trimethyl-2-oxocyclohexa-3-enyl-1(S)-oxycarbonylmethyl}ureido) butyrate

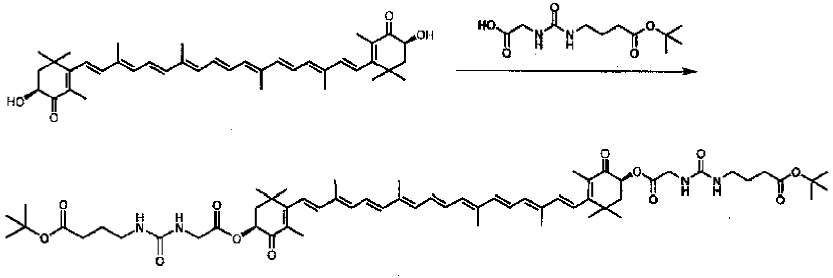

To 3(S),3' (S) -astaxanthin (1.8 kg), t-butyl 4-(3-carboxymethylureide) butyrate (compound (3.2) above) (2.75 kg) were added N,N-dimethyl-4-aminopyridine (2.95 kg) and methylene chloride (71.6 kg) and the mixture was stirred to prepare a solution. To the solution was added 1-(3-dimethylaminopropyl)-3-ethylcarbodiimide hydrochloride (4.63 kg) at -5 to 5°C and the mixture was stirred at the same temperature for 4 hours. To the reaction mixture was added water (3.6 kg), and the mixture was stirred. Further, ethyl acetate (48.4 kg) was added thereto, and the mixture was stirred and concentrated under a reduced pressure. To the concentrated residue were added ethyl acetate (48.4 kg) and water (45 kg), the mixture was stirred, and then the organic layer was separated. The resulting solution was washed sequentially with aqueous hydrochloric acid (0.3 M, 46.4 kg) three times, 10% sodium chloride aqueous solution (45.9 kg), aqueous sodium bicarbonate (about 7%, 48.2 kg), 20% sodium chloride aqueous solution (45 kg), dried over anhydrous magnesium sulfate, and concentrated under a reduced pressure to obtain the title compound (concentrated residue, 3.26 kg, purity 98.1%).

The purity of the product was determined by using high performance liquid chromatography (column: YMC-TriartC18 ExRS manufactured by YMC Co., Ltd., mobile phase: acetonitrile containing 0.025% trifluoroacetic acid/0.025% trifluoroacetic acid water = 30 to 98/70 to 2, flow rate: 1 mL/min, detection wavelength: 474 nm)
NMR spectrum (δ ppm, CDCl₃): 6.18-6.72 (14H, m), 5.56 (2H, dd, J=6.4, 13.2 Hz), 5.04 (2H, t, J=5.3 Hz), 4.81 (2H, t, 5.7 Hz), 4.25 (2H, dd, J=18.1, 6.6 Hz), 4.03 (2H, dd, J=18.3, 4.6 Hz), 3.19-3.26 (4H, m), 2.29 (4H, t, J=7.3 Hz), 2.02-2.13 (4H, m), 1.99 (12H, s), 1.90 (3H, s), 1.76-1.83 (4H, m), 1.44 (18H, s), 1.34 (6H, s), 1.23 (6H, s).
Mass spectrum (+ESI, m/z): 1081.88 (M+H)⁺, 1103.67 (M+Na)⁺.

### (3.4) Synthesis of 4-(3-{4-[18-(4(S)-[3-(3-carboxypropyl)ureidoacetoxy]-2,6,6-trimethyl-3-oxocyclohexa-1-enyl)-3,7,12,16-tetramethyloctadeca-1(E), 3(E), 5(E), 7(E), 9(E), 11(E), 13(E), 15(E), 17(E)-nonaenyl]-3,5,5-trimethyl-2-oxocyclohexa-3-enyl-1(S)-oxycarbonylmethyl}ureido)butyric acid:

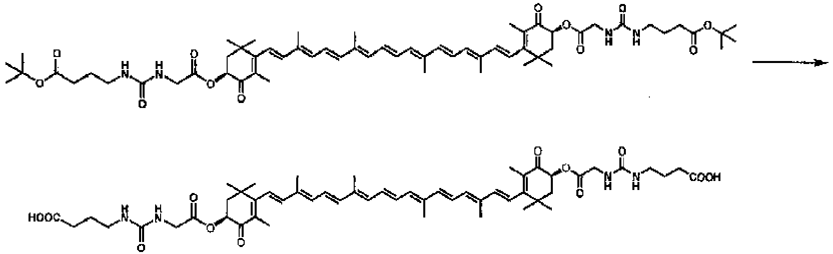

To t-butyl 4-(3-{4-[18-(4(S)-[3-(3-t-butoxycarbonylpropyl)ureidoacetoxy]-2,6,6-trimethyl-3-oxocyclohexa-1-enyl)-3,7,12,16-tetramethyloctadeca-1(E),3(E),5(E),7(E),9(E),11(E),13(E),15(E),17(E)-nonaenyl]-3,5,5-trimethyl-2-oxocyclohexa-3-enyl-1(S)-oxycarbonylmethyl}ureido)butyrate (compound (3.3) above, 18.12 g) was added formic acid (272 mL) and the mixture was stirred at 25 to 35°C for 1 hour. The reaction mixture was added to water (1087 mL), the mixture was stirred, ethyl acetate (1087 mL) was added thereto, the mixture was stirred and then the organic layer was separated. The organic layer was washed with water (543 mL) twice and with 10% brine (543.4 g) twice sequentially, dried over anhydrous magnesium sulfate and concentrated under a reduced pressure. The concentrated residue was dissolved in tetrahydrofuran (81.1 mL), water (8.11 mL), and to the resulting solution was added acetonitrile (486.8 mL) dropwise, and after the precipitation of solid was confirmed, the mixture was stirred for 1 hour. The precipitated solid was collected by filtration and dried to give the title compound (4.48 g, 90.7% purity) as a dark purple to dark red solid.

The purity of the product was determined using high performance liquid chromatography (column: YMC-Triart C18 ExRS manufactured by YMC Co., Ltd., mobile phase: acetonitrile containing 0.025% trifluoroacetic acid/0.025% trifluoroacetic acid water = 30 to 98/70 to 2, flow rate: 1 mL/min, detection wavelength: 474 nm)

### (3.5) Synthesis of 4-(3-{4-[18-(4(S)-[3-(3-carboxypropyl)ureidoacetoxy]-2,6,6-trimethyl-3-oxocyclohexa-1-enyl)-3,7,12,16-tetramethyloctadeca-1(E),3(E),5(E),7(E),9(E),11(E),13(E),15(E),17(E)-nonaenyl]-3,5,5-trimethyl-2-oxocyclohexa-3-enyl-1(S)-oxycarbonylmethyl}ureido)butyric acid:

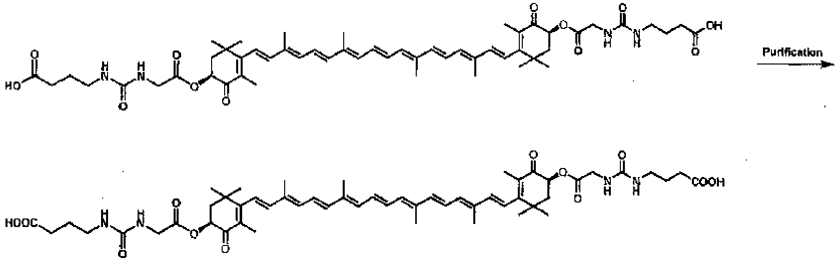

To 4-(3-{4-[18-(4(S)-[3-(3-carboxypropyl)ureidoacetoxy]-2,6,6-trimethyl-3-oxocyclohexa-1-enyl)-3,7,12,16-tetramethyloctadeca-1(E), 3(E), 5(E), 7(E), 9(E), 11(E), 13(E), 15(E), 17(E)-nonaenyl]-3,5,5-trimethyl-2-oxocyclohexa-3-enyl-1(S)-oxycarbonylmethyl}ureido)butyric acid (crude product of (3.4) above) (4.0 g) were added tetrahydrofuran (18.4 mL) and water (2.0 mL), and the mixture was stirred to dissolve the above compound. Acetonitrile (60 mL) was added dropwise to the solution, and after the precipitation of a solid was confirmed, the mixture was stirred for 1 hour. The precipitated solid was collected by filtration and dried to obtain a dark purple to dark red solid (3.31 g, 96.9% purity). To the obtained solid (3.26 g), tetrahydrofuran (15.0 mL) and water (1.6 mL) were added, and the mixture was stirred to dissolve the solid. Acetonitrile (49 mL) was added dropwise to the solution, and after the precipitation of a solid was confirmed, the mixture was stirred for 1 hour. The precipitated solid was collected by filtration and dried to obtain a dark purple to dark red solid (2.93 g, 98.9% purity). To the resulting solid (2.38 g) were added tetrahydrofuran (10.9 mL) and water (1.2 mL), and the mixture was stirred to dissolve the solid. Acetonitrile (36 mL) was added dropwise to the solution, and after the precipitation of a solid was confirmed, the mixture was stirred for 1 hour. The precipitated solid was collected by filtration and dried to give the title compound (2.18 g, 99.3% purity) as a dark purple to dark red solid.

The purity of the product was determined by using high performance liquid chromatography (column: YMC-Triart C18 ExRS manufactured by YMC Co., Ltd., mobile phase: acetonitrile containing 0.025% trifluoroacetic acid/0.025% trifluoroacetic acid water = 30 to 98/70 to 2, flow rate: 1 mL/min, detection wavelength: 474 nm)

### (3.6) Synthesis of 4-(3-{4-[18-(4(S)-[3-(3-carboxypropyl)ureidoacetoxy]-2,6,6-trimethyl-3-oxocyclohexa-1-enyl)-3,7,12,16-tetramethyloctadeca-1(E),3(E),5(E),7(E),9(E),11(E),13(E),15(E),17(E)-nonaenyl]-3,5,5-trimethyl-2-oxocyclohexa-3-enyl-1(S)-oxycarbonylmethyl}ureido)butyric acid dilysine salt; Compound X:

To 4-(3-{4-[18-(4(S)-[3-(3-carboxypropyl)ureidoacetoxy]-2,6,6-trimethyl-3-oxocyclohexa-1-enyl)-3,7,12,16-tetramethyloctadeca-1(E),3(E),5(E),7(E),9(E),11(E),13(E),15(E),17(E)-nonaenyl]-3,5,5-trimethyl-2-oxocyclohexa-3-enyl-1(S)-oxycarbonylmethyl}ureido)butyric acid (the compound (3.5) above) (0.50 g), ethanol (10 mL), and water (0.5 mL) were added and the mixture was stirred. To the suspension solution was added a solution of L-lysine monohydrate (0.174 g,) in water (2 mL) at a room temperature. To the reaction mixture was added water (7.5 mL), and the mixture was stirred to dissolve the mixture. Ethanol (32 mL) was added dropwise to the reaction mixture at a room temperature, and after the precipitation of a solid was confirmed, the mixture was stirred for 1 hour. The precipitated solid was collected by filtration and dried to give the title compound (0.47 g, 98.6% purity, 99.0%de optical purity) as a dark purple to dark red solid.

The purity of the product was determined by using high performance liquid chromatography in the same manner as described above. The optical purity was determined using high-speed liquid chromatography (column: YMC CHIRAL ART Amylose-SA (5 µm, .4.6 mml.D. x 250 mm) manufactured by YMC Co., Ltd., column temperature: 25°C and mobile phase: THF/water/TFA (40: 60: 0.1), flow rate: 1 mL/min, detection wavelength: 474 nm, column retention time: 15.4 minutes (S, S), 17.6 minutes (meso), 20.6 minutes (R, R)).

## Claims

1. A colitis ameliorating agent comprising a trans-astaxanthin derivative of formula (I), geometric isomers thereof, a mixture of the geometric isomers, an optical isomer thereof, or a salt thereof; 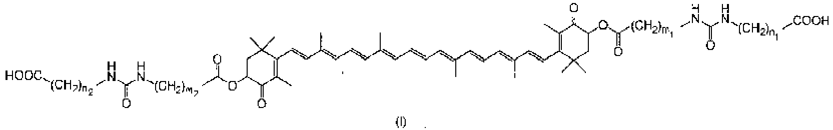 (wherein m₁, m₂, n₁ and n₂ are each the same or different and mean an integer of 1 to 6).

2. The colitis ameliorating agent according to claim 1, wherein in formula (1), m₁ and m₂ are each an integer of 1, and n₁ and n₂ are each an integer of 3.

3. The colitis ameliorating agent according to claim 1 or 2, wherein the salt is a lysine salt.

4. A colitis ameliorating agent comprising an optically active trans-astaxanthin derivative of formula (IA), geometric isomers thereof, a mixture of the geometric isomers, or a salt thereof; 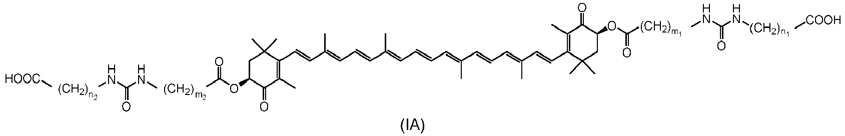 (wherein m₁, m₂, n₁ and n₂ are each the same or different and mean an integer of 1 to 6).

5. The colitis ameliorating agent according to claim 4, wherein in formula (IA), m₁ and m₂ are each an integer of 1, and n₁ and n₂ are each an integer of 3.

6. The colitis ameliorating agent according to claim 4 or 5, which is substantially free of an optically active cis-astaxanthin derivative and a salt thereof corresponding to the optically active trans-astaxanthin derivative of formula (IA).

7. A colitis ameliorating agent comprising a high-purity optically active trans-astaxanthin derivative or a salt thereof according to any one of claims 4 to 6.

8. The colitis ameliorating agent according to any one of claims 1 to 7, wherein the colitis is ulcerative colitis and or Crohn's disease of colon.

9. Use of a trans-astaxanthin derivative of formula (I), geometric isomers thereof, a mixture of the geometric isomers, an optical isomer thereof, or a salt thereof for producing a colitis ameliorating agent; 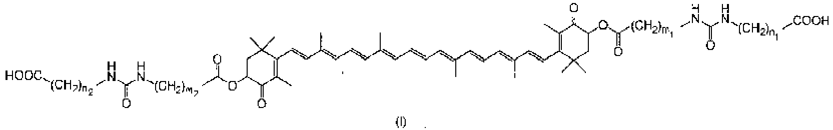 (wherein m₁, m₂, n₁ and n₂ are each the same or different and mean an integer of 1 to 6).

10. A trans-astaxanthin derivative of formula (I), geometric isomers thereof, a mixture of the geometric isomers, an optical isomer thereof, or a salt thereof for ameliorating colitis; 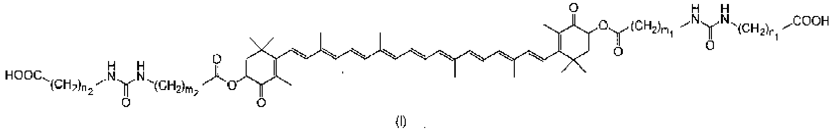 (wherein m₁, m₂, n₁ and n₂ are each the same or different and mean an integer of 1 to 6).

11. A therapeutic method for ameliorating colitis comprising administering an effective amount of a trans-astaxanthin derivative of formula (I), geometric isomers thereof, a mixture of the geometric isomers, an optical isomer thereof, or a salt thereof, 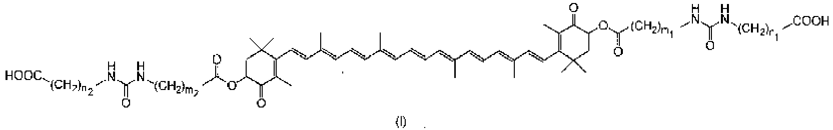 (wherein m₁, m₂, n₁ and n₂ are each the same or different and mean an integer of 1 to 6).
